# EUROPÄISCHE PATENTSCHRIFT

(11) **EP 3 445 233 B1**
(45) Veröffentlichungstag und Bekanntmachung des Hinweises auf die Patenterteilung: **06.11.2024**
(21) Anmeldenummer: 16836147.5
(22) Anmeldetag: 07.12.2016
(51) Int. Cl.: A61B 5/02, G06N 3/08, A61B 5/00, A61B 5/022, A61B 5/021

(54) **VERFAHREN ZUR ERFASSUNG VON ARTERIELLEN PARAMETERN EINES MENSCHEN UND VORRICHTUNG ZUR DURCHFÜHRUNG DES VERFAHRENS**
METHOD FOR DETECTING ARTERIAL PARAMETERS OF A HUMAN BEING AND DEVICE FOR CARRYING OUT SAID METHOD
PROCÉDÉ D'OBTENTION DE PARAMÈTRES ARTÉRIELS HUMAINS ET DISPOSITIF POUR LA MISE EN OEUVRE DE CE PROCÉDÉ

(30) Priorität: 08.12.2015 DE 102015015784
(43) Veröffentlichungstag der Anmeldung: 27.02.2019
(73) Patentinhaber: Wsh Engineering Services GBR (Vertretungsberechtigter Gesellschadter: Hieronymi, Andreas), 61440 Oberursel (DE)
(72) Erfinder: SCHUMACHER, Gerhard, 35510 Butzbach (DE)
(74) Vertreter: Körner, Volkmar Horst
(86) Internationale Anmeldenummer: PCT/DE2016/000429
(87) Internationale Veröffentlichungsnummer: WO 2017/097281

(56) Entgegenhaltungen:
- WO-A1-2009/029386
- US-A- 5 680 866
- US-A1- 2006 235 323
- US-A1- 2010 121 204
- US-A1- 2010 274 102
- WESTERHOF N ET AL: "Analog studies of the human systemic arterial tree", JOURNAL OF BIOMECHANICS, PERGAMON PRESS, NEW YORK, NY, US, vol. 2, no. 2, 1 May 1969 (1969-05-01), pages 121, XP026262071, ISSN: 0021-9290, [retrieved on 20211117], DOI: 10.1016/0021-9290(69)90024-4

## Beschreibung

Die Untersuchung hämodynamischer Eigenschaften des menschlichen Gefäßsystems erfolgt meist pulskonturbasiert unter Bestimmung und Auswertung charakteristischer Merkmale der Verlaufskurve wie beispielsweise Punkte bestimmter Steigung oder Krümmung. Oft werden dabei Surrogatparameter wie z.B. die Pulskurvengeschwindigkeit als Maß für die generelle Steifigkeit der Arterien oder die Auswurfdauer als Maß für die Leistungsfähigkeit des Herzens aus der Lage dieser charakteristischen Punkte wie z.B. dem Beginn der Erregungswelle, deren Maximum sowie der Inzisur ermittelt und zur Diagnose kardiovaskulärer Krankheiten herangezogen. Direkte Messung auch solcher Surrogatparameter sind nur invasiv möglich, während die nicht-invasive Erfassung solcher Parameter immer durch die direkte Gefäßumgebung (Muskel- und Fettgewebe bzw. Knochenmasse) behindert wird und teilweise einer Korrektur unterzogen werden muss.

Die US 2006 0235323 A1 offenbart ein Verfahren zur Bestimmung eines kardiovaskulären Parameters, der einem Herzzeitvolumen entspricht oder sich von diesem ableiten lässt. Hierzu wird ein aktueller Druckwellenformdatensatz, der dem arteriellen Blutdruck über einen aktuellen Druckzyklus entspricht, eingegeben und definierte Parameter einer angenommenen Eingangsflusswellenform bestimmt.

Die US 2010 0274102 A1 offenbart ein Verfahren zur Verarbeitung von Pulsoximeterdaten unter Verwendung eines dynamischen Zustandsraummodells zum Extrahieren eines geschätzten Wertes für mindestens einen nicht durch das Pulsoximeter gemessenen physiologischen Parameter. Die Literaturstelle Westerhof et al: "Analog studies of the human systemic arterial tree", Jornal of Biomechanics, Pergamon Press, New York, NY, US, Bd.2. Nr. 2, 1. Mai 1969 (1969-05-01), Seite 121, XP026262071, ISSN: 0021-9290, DOI: 10.1016/0021-9290(69)90024-4 offenbart ein Arterienmodell mit hintereinander geschalteten RLC-Schwingkreisen als Windkessel-Elemente. In dieser Schrift werden die ursprünglich auf cm-Abschnitten basierenden Arterienelemente zu Bereichen gleicher Arterienabschnitte zusammenfasst und Ersatzwerte für R,L und C gelegt, die für die direkte Reihenschaltung von Widerständen und Induktivitäten bzw. die Parallelschaltung von Kondensatoren gelten, nicht jedoch für die Aneinanderreihung von RLC-Schwingkreisen.

Bei Anwendung dieser pulskonturbasierten Verfahren erfolgt jedoch meist keine eindeutige Trennung zwischen der Compliance der Arterien, dem Leitungswiderstand, der dem Blutfluss entgegensteht, und der Fließfähigkeit des Blutes. Unter Fließfähigkeit verstehen wir eine Kombination aus Massenträgheit, Viskosität und ähnlicher Größen des Blutes. Zur Verbesserung der Diagnose und Behandlung kardiovaskulärer Krankheiten sowie krankhafter Veränderungen wie beispielsweise Bluthochdruck, Arteriosklerose, Stenosen, Aneurysmen oder Herzinsuffizienz ist es jedoch wünschenswert, eine zuverlässige Unterscheidung hinsichtlich der Gefäß-Compliance, des Gefäß-Leitungswiderstandes und der Fließfähigkeit des Bluts zu treffen, da sich diesen Eigenschaften spezifische Krankheitsbilder zuordnen lassen. So geht beispielsweise generell verminderte arterielle Compliance mit altersbedingtem Bluthochdruck einher, während erhöhter Leitungswiderstand ein Indiz für athero- und/oder arterio-sklerotische Veränderungen ist und verminderte Fließfähigkeit des Bluts beispielsweise auf eine erhöhte Herzbelastung oder erhöhtes Schlaganfall-Risiko hinweisen kann. Die Messung der Fließfähigkeit des Bluts erfolgt bisher üblicherweise durch Bestimmung des Hämatokritwertes von Blutproben in Zentrifugen.

Lösungen zur oszillometrischen Messung arterieller Compliance werden z.B. in US 5,447,163 und US 6,733,461 beschrieben. Hierzu sind jedoch meist umständliche Kalibrierungen und Rekalibrierungen erforderlich. Die daraus resultierenden Messunsicherheiten und eingeschränkte Reproduzierbarkeit der Messergebnisse können jedoch den verbreiteten Einsatz entsprechender Technologien behindern. Andere Verfahren zur Messung vaskulärer Impedanzen, wie beispielsweise in US 5,211,177 beschrieben, stützen sich auf Berechnungen, die auf dem sogenannten Windkesselmodell basieren, setzen hierzu jedoch die Messung weiterer physiologischer Parameter, wie z.B. des Herzzeitvolumens und des Mittleren Arteriellen Drucks (MAP), voraus und arbeiten dabei ebenfalls pulskonturbasiert, d.h. unter Bestimmung und Auswertung charakteristischer Merkmale der Verlaufskurve. Wer mit der visuellen Abschätzung oder automatischen Detektion solcher charakteristischen Merkmale vertraut ist, wird zustimmen, dass es häufig Fälle gibt, in denen die exakte Position und Amplitude, z.B. aufgrund der Überlagerung von Erregungswelle und reflektierter Welle, nicht eindeutig bestimmt werden können. Daher sind solche Messergebnisse im Falle wiederholter Messungen zum Teil hohen Schwankungen unterworfen.

Die vorliegende Erfindung beschreibt ein Verfahren und eine Vorrichtung zur Durchführung des Verfahrens, die eine Ermittlung der arteriellen Compliance, des arteriellen Leitungswiderstandes und der Massenträgheit des Blutes zuverlässig und reproduzierbar unter Vermeidung der oben aufgeführten Nachteile erlauben.

Das erstgenannte Problem wird erfindungsgemäß durch ein Verfahren nach Anspruch 1 gelöst. Weitere Ausführungsbeispiele finden sich in abhängigen Ansprüchen 2-4.

Die dem Simulationsmodell des Menschen zuzuführenden Parameter könnten beispielsweise aus der Pulskurve iterativ bestimmt werden. Das Verfahren lässt sich jedoch gemäß einer vorteilhaften Weiterbildung der Erfindung deutlich beschleunigen, wenn die an dem Menschen gemessene Pulskurve einem geeigneten, trainierten neuronalen Netzwerk zugeführt wird und dieses die gesuchten Parameter mit Hilfe des in ihm gespeicherten Wissens über die Beziehungen zwischen Pulskurvenform und der zugehörigen Parameter ausgibt. Die berechneten Parameter lassen sich anschließend im Simulationsmodell des Menschen verwenden, um Pulskurven an verschiedenen Stellen des simulierten Arterienbaums zu untersuchen.

Besonders genaue Parameter zur arteriellen Gefäßsteifigkeit oder Gefäß-Impedanz lassen sich gemäß einer vorteilhaften Weiterbildung der Erfindung einfach schaffen, wenn die Pulskurve gemessen wird, indem der Manschettendruck bis zum Blutflussstillstand durch Okklusion der Arterien gesteigert wird, wenn dieser Manschettendruck eine vorgegebene Anzahl an Herzzyklen aufrechterhalten wird und wenn der Druckverlauf mehrerer Pulskurven in der Manschette gemessen wird und wenn aus dem Druckverlauf mehrerer Pulskurven eine gemittelte Pulskurve erzeugt wird. Damit kann die Ermittlung der Pulskurvengeschwindigkeit im Bereich der Aorta, des Aorta-Blutdrucks (auch Zentraldruck genannt), des Augmentations-Index oder der linksventrikulären Auswurfdauer durch kurzzeitiges vollständiges Verschließen, beispielsweise der Handgelenk-Arterien ermöglicht werden. Durch diese vollständige Okklusion können die ermittelten Pulskurven in ausreichendem Maße von störenden Einflüssen lokaler Blutflussturbulenzen und distaler Reflektionen, die beide zu verfälschten Messergebnissen führen können, befreit werden.

Das zweitgenannte Problem, nämlich die Schaffung einer Vorrichtung zur Durchführung des Verfahrens wird erfindungsgemäß durch eine Vorrichtung nach Anspruch 5 gelöst. Weitere Ausführungsbeispiele finden sich in abhängigen Ansprüchen 6-8.

Eine besonders schnelle Bestimmung der Gefäß- und Kreislaufparameter lässt sich gemäß einer anderen vorteilhaften Weiterbildung der Erfindung erreichen, wenn die Berechnungseinheit anstelle des Optimierungsalgorithmus ein Neuronales Netzwerk ist.

Durch diese Gestaltung erkennt das Neuronale Netzwerk das Muster der eingegebenen Pulskurve und kann die Parameter direkt ausgeben. Eine derartige Mustererkennung ist in der Praxis deutlich schneller als ein Optimierungsalgorithmus zur Bestimmung der Parameter.

Das Simulationsmodell des Menschen ermöglicht gemäß einer anderen vorteilhaften Weiterbildung der Erfindung einen Rückschluss einer am Arm oder Bein gemessenen Pulskurve auf arterielle Parameter des Menschen, wenn im Simulationsmodell des menschlichen Körpers die Arterien in einzelne Arterienabschnitte unterteilt sind und die Arterienabschnitte als elektrisches Ersatzschaltbild mittels Widerständen, Kapazitäten und Induktivitäten angenähert sind. Damit bildet das ein Arterienmodell nachbildende elektro-hydraulische Simulationsmodell ein Analogon, mit dem über eine nichtinvasive Messung an Arm oder Bein bis zum Zentraldruck, der Aortensteifigkeit und dem Aorta-Blutfluss zurückgerechnet werden kann.

Die in-vivo-Erfassung der Verlaufskurve eines hämodynamischen Vorgangs gestaltet sich gemäß einer vorteilhaften Weiterbildung der Erfindung besonders einfach, wenn eine Vorrichtung zur Messung der Pulskurve eine zur Anlage an dem menschlichen Körper vorgesehene Druckmanschette und eine Druckquelle zur Erzeugung eines zum Blutflussstillstand durch Okklusion der Arterien, insbesondere den Handgelenk-Arterien, ermöglichenden Drucks und zur Aufrechterhaltung dieses Drucks für eine vorgesehene Anzahl an Herzzyklen ausgebildet ist.

### Beschreibung der Zeichnungen

Fig. 1 zeigt das elektrische Ersatzschaltbild eines Windkesselelements
Fig. 2 zeigt ein elektro-hydraulisches Arterienmodell (Analogon), Quelle: [1]
Fig. 3 zeigt einen möglichen Amplitudenverlauf der elektrischen Quelle eines Analogons
Fig. 4 zeigt die Präsentation von Amplituden an den Eingängen eines erfindungsgemäßen Neuronalen Netzes bei dessen Training
Fig. 5 zeigt die Komponenten einer Vorrichtung zur Messung arterieller Parameter
Fig. 5a zeigt eine Übersichtabbildung zum erfindungsgemäßen Verfahren
Fig. 6 zeigt die Darstellung der arteriellen Parameter in einem Radar-Diagramm
Fig. 7 zeigt ein Beispiel für eine Simulation einer in-vivo ermittelten Verlaufskurve

### Beschreibung der Erfindung

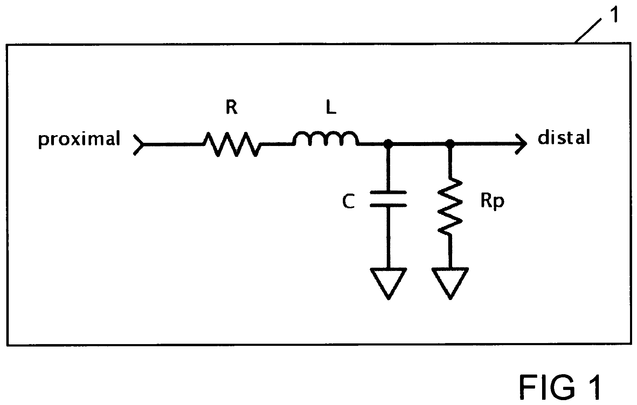

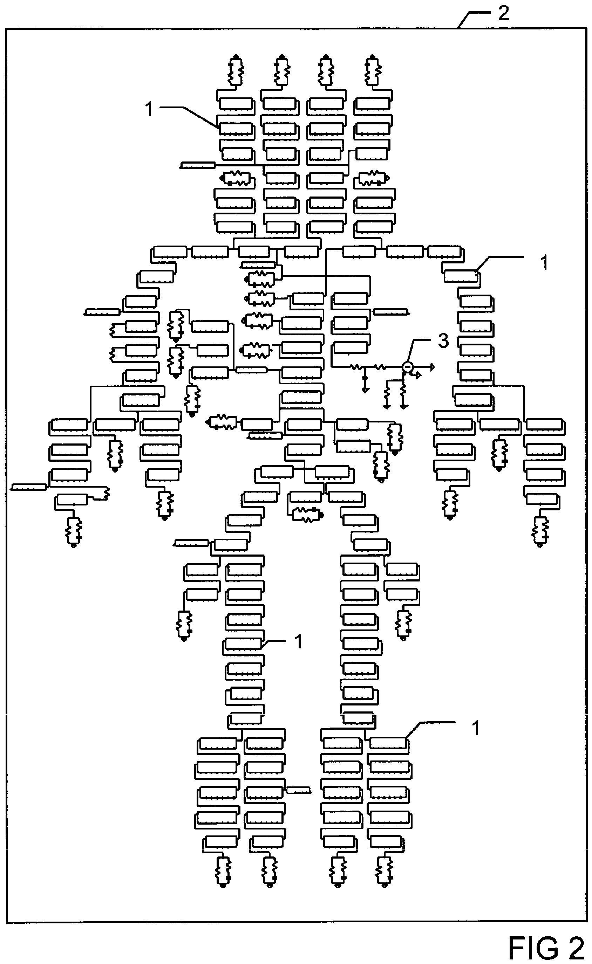

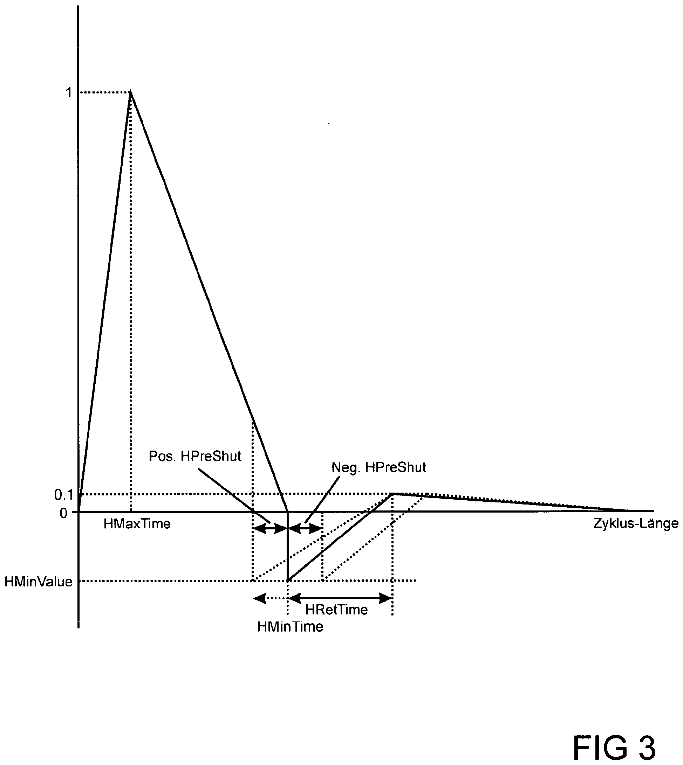

Es ist bereits bekannt, dass sich Fluss- und Druckverhältnisse im arteriellen Gefäßsystem mit Hilfe sogenannter elektro-hydraulischer Analoga künstlich nachbilden lassen. Dabei werden mehrere sogenannte Windkesselelemente (Fig. 1) aneinandergereiht. Durch die Reihenschaltung mehrerer dieser aus Kapazitäten C, ohmschen Widerständen R und Induktivitäten L bestehenden Windkesselelemente (Fig.1) und der Beaufschlagung des Eingangs der Reihenschaltung mit einer elektrischen Strom- oder Spannungsquelle mit Verläufen entsprechend Fig.3, die die Pumpfunktion des Herzens nachbildet, können Fluss- oder Druckwerte an beliebigen Stellen des somit gebildeten Körpermodells, im Folgenden Analogon (2), (Fig. 2) genannt, errechnet werden. Die Kapazitäten C, Widerstände R und Induktivitäten L der einzelnen Windkesselelemente (Fig. 1) repräsentieren die arterielle Compliance (C), den arteriellen Leitungswiderstand (R) und die Fließfähigkeit des Bluts (L) im betrachteten Gefäßabschnitt. Die berechneten elektrischen Ströme repräsentieren dabei den arteriellen Blutfluss, die elektrischen Spannungen den arteriellen Druck.

Zweck des beschriebenen Gefäßmodells ist die Simulation hämodynamischer Vorgänge. Der vorliegenden Erfindung liegt jedoch die Umkehrung dieses Prinzips zu Grunde: Aus vorhandenen, hämodynamischen Verläufen sollen die medizinisch maßgeblichen arteriellen Parameter Compliance, Leitungswiderstand und Fließfähigkeit des Bluts bestimmt werden. Zur Lösung der erfindungsgemäßen Aufgabenstellung wird daher zunächst ein elektro-hydraulisches Arterienmodell als Simulationsmodell erstellt. Dieses Simulationsmodell wird auch Analogon genannt. Ein solches, sogenanntes elektro-hydraulisches Analogon (2) kann z.B. mittels computerunterstützter Analyse elektrischer Netzwerke, wie sie unter dem Begriff SPICE (*Simulation Program with Integrated Circuit Emphasis*) geläufig ist, ohne großen apparativen Aufwand erstellt werden. Daraus können Ströme und Spannungen an beliebigen Stellen des Modells berechnet werden.

Es ist auch bekannt, dass auf dieser Technik basierende Modellbildungen fehlerbehaftet sein können, da sie, aufgrund ihrer Komplexität, zwangsläufig vereinfachende Annahmen enthalten. So erfolgt beispielsweise bei der Modellbildung eine künstliche Einteilung in einzelne Arteriensegmente, wohingegen im Körper von Lebewesen eine kontinuierliche Veränderung des Durchmessers der Arterien und ihrer Elastizität gegeben ist. Des Weiteren bleiben auf schwankendem Druck basierende, nichtlineare Zusammenhänge unberücksichtigt. Die mit dieser Technik erzielbaren Simulationsergebnisse zeigen u.a. deshalb immer wieder Abweichungen von in-vivo ermittelten hämodynamischen Verläufen. Beispielsweise ist den Erfindern aufgefallen, dass zunächst die C-, R- und L-Werte für 121 Windkesselelemente (Fig. 1) mit arteriellen Segmentlängen von 1 cm ermittelt wurden, bei der anschließenden Simulation aber, aufgrund des zum damaligen Zeitpunkt zur Nachbildung des Analogons 2 erforderlichen hohen apparativen Aufwands, mehrere (bis zu 8) dieser Windkesselelemente (Fig. 1) durch Bildung von Parallelersatz-Kapazitäten sowie Reihenersatz-Widerständen und -Induktivitäten zusammengefasst wurden. Der hierbei aufgrund der Vernachlässigung des dynamischen Schwingungsverhaltens der einzelnen Windkesselelemente (Fig. 1) entstehende Fehler führt zu deutlichen Abweichungen der simulierten Ergebnisse gegenüber realen hämodynamischen Verhältnissen, weshalb im erfindungsgemäßen Ausführungsbeispiel des Analogons 2 anstelle der 121 Segmente gemäß nun 711 Segmente mit auf 1 cm Segmentlänge zurückgerechneten R, L und C Werten berücksichtigt werden. Berücksichtigt man, dass der Einfluss des Korrekturnetzwerks, bestehend aus R₀ und R₂ bis Rₘ sowie L₂ bis Lₘ, auf das Simulationsergebnis gering ist, erhält man das in Fig. 1 dargestellte Windkesselelement. Zusätzlich wurden empirisch ermittelte Korrekturfaktoren eingeführt, um die hämodynamischen Pulskurvenverläufe möglichst exakt nachbilden zu können. Die Korrekturfaktoren lagen im Bereich zwischen 0,3 und 1,0 und wurden für den Arm berechnet. Aus der wissenschaftlichen Literatur sind verschiedene Windkesselmodelle bekannt, die sich in Anzahl und Anordnung der eingesetzten Kapazitäten C, Widerstände R und Induktivitäten L sowie in ihren Vor- und Nachteilen unterscheiden. Verschiedene, von dem in Fig. 1 als Ausführungsbeispiel gezeigten Windkesselelement (Fig. 1) und dem in Fig. 2 beispielhaft dargestellten Analogon (2) abweichende Ausprägungen sind daher im Sinne dieser Erfindung ebenfalls zur Lösung der Aufgabenstellung denkbar. Dies betrifft z.B. Anzahl, Wert und Position der Komponenten jedes einzelnen Windkesselements (Fig. 1), genannt seien hier exemplarisch 2-, 3- oder 4-Elementsowie symmetrische und unsymmetrische Windkesselmodelle, sowie die spezifische Anordnung und Kombination der einzelnen Windkesselelemente (Fig. 1) innerhalb des Analogons (2).

An einem geeigneten Analogon (2) müsste das Simulationsergebnis an einem bestimmten Gefäßsegment theoretisch mit dem Verlauf einer in-vivo am gleichen Gefäßabschnitt ermittelten Verlaufskurve übereinstimmen. In der Praxis existieren jedoch oft Abweichungen zwischen beiden Verlaufskurven. Diese Abweichungen basieren auf zuvor beschriebenen Modellmängeln, auf individuellen, generellen in-vivo-Abweichungen der arteriellen Compliance, des Leitungswiderstands oder der Fließfähigkeit des Bluts von den Durchschnittswerten des Modells sowie auf Abweichungen der Werte einzelner Segmente, z.B. durch Stenosen und Aneurysmen. Abweichungen, die auf solche krankhaften Veränderungen zurückzuführen sind, sind vermeidbar, wenn an Bereichen gemessen wird, wo solche Veränderungen selten auftreten, wie z.B. an den Arm-Arterien.

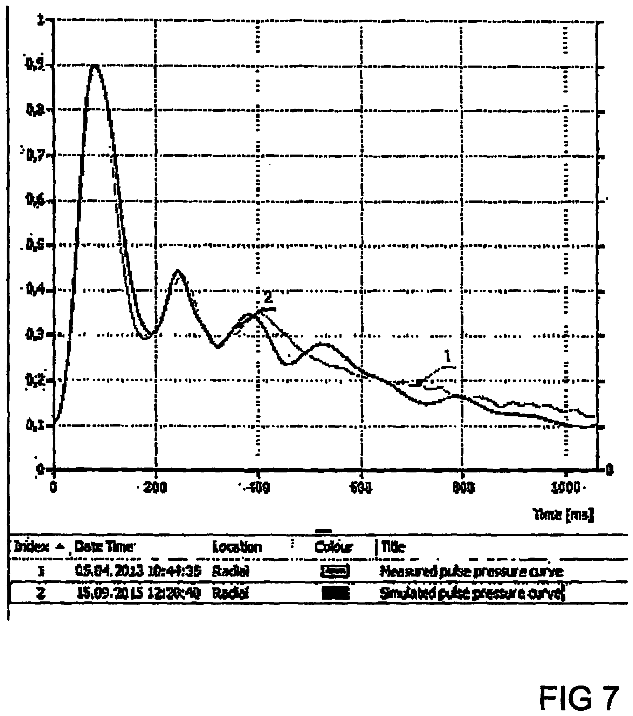

Die zuvor beschriebenen individuellen, generellen Abweichungen der arteriellen Compliance, des Leitungswiderstands oder der Fließfähigkeit des Bluts kommen dadurch zustande, dass die Komponentenwerte für C, R1 und L1 für einen gesunden Durchschnittsprobanden mit einer Körpergröße von 175 cm, einem Gewicht von 75 kg etc. berechnet wurden. Zur Ermittlung der erfindungsgemäß zu bestimmenden, individuell unterschiedlichen, arteriellen Parameter Compliance, Leitungswiderstand und Fließfähigkeit wird daher stellvertretend für jeden dieser Parameter ein zusätzlicher Korrekturfaktor α(C), α(R) und α(L) eingeführt. Zur Simulation der hämodynamischen Verläufe mit dem Analogon (2) wird jeder der Komponentenwerte C, R1 und L1 mit seinem Korrekturfaktor multipliziert. Die Korrekturfaktoren α(C), α(R) und α(L) nehmen dabei, im Gegensatz zu den in [1] angeführten segmentspezifischen Komponentenwerten C, R1 und L1 für alle Segmentabschnitte den gleichen Wert an. Durch manuelle Einstellung der drei Korrekturfaktoren α(C), α(R), α(L) lässt sich nun das Simulationsergebnis so adjustieren, dass die zuvor beschriebenen individuellen Abweichungen verschwinden und das Simulationsergebnis mit der in-vivo ermittelten Verlaufskurve übereinstimmt (Fig. 7). Bei der Auswertung der Messergebnisse einer Vielzahl von Probanden zeigte sich, dass ein gewisser Bereich für alle Korrekturfaktoren existiert. Für die graphische Darstellung dieser individuellen Korrekturfaktoren wird weiter unten ein so genanntes Radardiagramm vorgeschlagen. Zu diesem Zweck wurde der jeweilige Bereich auf 100% skaliert, wodurch die Vergleichbarkeit der Korrekturdaten für alle Probanden gewährleistet ist. Der Einfluss von Abweichungen, die auf Modellmängeln basieren, wird durch Anwendung dieser Korrekturfaktoren ebenfalls verringert.

Um übereinstimmende Kurvenverläufe zu erhalten, ist es darüber hinaus noch erforderlich, das Verhalten der elektrischen Quelle (3) so nachzubilden, dass dies mit dem individuellen Verhalten des Herzens zum Zeitpunkt der in-vivo-Erfassung der hämodynamischen Verläufe übereinstimmt. In vorliegendem Ausführungsbeispiel wird dazu eine elektrische Quelle (3) verwendet, deren zeitlicher Verlauf der Ausgangsgröße dem Verlauf in Fig. 3 entspricht, wobei bei HMaxTime das Maximum der Ausgangsgröße (Druck- oder Flusskurve) erreicht wird. Der Zeitpunkt des Aortenklappenschlusses, HMinTime, ist im hämodynamischen Verlauf durch ein lokales Minimum mit einem Fluss der Amplitude HMinValue in umgekehrter Richtung gekennzeichnet und wird oft auch als Inzisur, linksventrikuläre Auswurfzeit oder Systolendauer bezeichnet und beträgt z.B. bei gesunden Menschen ca. 250 ms. Das Maximum der hämodynamischen Verlaufskurve, HMaxTime, ist bei Menschen im Normalfall bereits nach ca. 100 ms der Periodendauer eines gesamten Herzzyklus erreicht. Die Periodendauer des gesamten Herzzyklus, Cycle length, lässt sich an der vorliegenden in-vivo-Kurve ablesen und kann manuell in die Parametrisierung der Quelle (3) des Analogons (2) übertragen werden. Zur möglichst exakten Simulation realer Verhältnisse wird in diesem Beispiel zusätzlich ein zweites, positives Maximum, dessen Amplitude 10% des Hauptmaximums entspricht, zum Zeitpunkt HRetTime eingeführt. Zur weiteren Verfeinerung kann das Modell um einen HPreShut-Wert erweitert werden, der sowohl positiv als auch negativ (Fig. 3) sein kann und wodurch der Abfall der Erregungswelle variiert werden kann. Die einzelnen, geraden Segmente der in Fig. 3 dargestellten Amplitudenverläufe können zwecks originalgetreuerer Herznachbildung auch aus gekrümmten Kurvensegmenten bestehen. Dies kann im Analogon (2) beispielsweise annähernd durch Verwendung einer sogenannten abschnittweise-linearen Quelle (3) mit entsprechender Anzahl an Stützstellen oder kontinuierlich durch einen der Quelle (3) im Analogon (2) nachgeschalteten elektrischen Tiefpass erreicht werden.

Da die zuvor beschriebene, manuelle Ermittlung der Korrekturfaktoren α(C), α(R), α(L) und der Quellen-Parameter HMaxTime, HMinTime, HMinValue, HRetTime und HPreShut für eine individuelle in-vivo-Bestimmung zu aufwändig ist, soll nun ein erfindungsgemäßes Verfahren beschrieben werden, mit dem dies automatisiert werden kann. Dieses Verfahren kann in der Schaffung einer Software bestehen, die es gestattet auf direktem Wege die gesuchten Parameter zu ermitteln. Dieses Verfahren besteht in dieser Erfindung aus einem Genetischen Algorithmus, kann aber auch durch jeden anderen Optimierungs-Algorithmus realisiert werden. Da die Optimierungs-Algorithmen i.a. sehr zeitaufwändig sind, können die gesuchten Parameter auch mit Hilfe eines entsprechend trainierten neuronalen Netzes bestimmt werden. Das Training des in dem Verfahren beschriebenen Algorithmus beinhaltet zunächst die in-vivo-Erfassung einer ausreichenden Anzahl hämodynamischer Verlaufskurven (3). Bei diesen Verlaufskurven kann es sich um einen sogenannten Druck-, Volumen- oder Flusspuls handeln, da sich die beschriebene elektro-hydraulische Analogie auf diese Größen bezieht. In gleicher Weise können auch bereits vorhandene Kurven, z.B. aus Datenbanken, für das Training verwendet werden, sofern diese Kurven auf Größen basieren, auf die die elektro-hydraulische Analogie anwendbar ist.

In der Erfindung wird ein evolutionärer Optimierungs-Algorithmus verwendet, es können jedoch auch andere Optimierungsverfahren angewendet werden. Der hier verwendete evolutionäre Optimierungs-Algorithmus definiert als "Gene" die zu bestimmenden Modellparameter P₁ ... Pₙ. Die Gesamtheit der Gene kann als "Chromosom" bezeichnet werden. Der Algorithmus generiert zunächst eine Anfangspopulation der Größe 30, die aus einzelnen "Individuen" mit jeweils zufallsbestimmten Chromosomen besteht. Zu diesen 30 Individuen wird jeweils mit Hilfe des Simulationsmodells die resultierende simulierte Pulskontur bestimmt. Eine "Fitnessfunktion" bestimmt anschließend zu jedem Individuum die Summe der Fehlerquadrate zwischen der gemessenen Pulskontur und der Pulskontur des Individuums. Damit kann die Population gemäß der Fitness, also der Güte der Simulationen, sortiert werden. Anschließend wird die folgende Sequenz durchlaufen, bis ein Abbruchkriterium erfüllt ist:
- Selektion: Auswahl der besten 40% der Individuen für die Rekombination
- Rekombination: Kombination von Teilen der Chromosomen der ausgewählten Individuen und damit Erzeugung von Nachfahren
- Mutation: Zufällige Veränderung der Nachfahren
- Evaluation: Bestimmung der Fitness der Nachfahren
- Einsortierung der neuen Nachfahren (der neuen "Generation") in die Population gemäß der Güte ihrer Simulationen.

Als Abbruchkriterium wird eine vorher festgelegte Fitness, also eine festgelegte Summe der Fehlerquadrate zwischen der Simulationskontur des besten Individuums und der gemessenen Pulskontur verwendet. Zusätzlich wird für den Fall, dass die gemessene Pulskontur aufgrund starker Artefakte keine hohe Übereinstimmung mit dem besten Individuum erreichen kann, als Abbruchkriterium eine festgelegte Anzahl von Generationen herangezogen, die seit der letzten Verbesserung der Population erzeugt wurden, ohne dass ein neues, verbessertes Individuum entstand.

Ein wesentlicher Vorteil der vorliegenden Erfindung ist, dass das Analogon (2) an die Spezifika verschiedener Messmethodiken anpassbar ist. So kann beispielsweise für suprasystolische Brachialmessungen, bei denen die Erfassung des hämodynamischen Verlaufs unter Vollokklusion der Brachialarterie erfolgt, ein Abschlusswiderstand ∞ (Unterbrechung) hinter dem Segment der Brachialarterie (s. Fig. 2) platziert werden, bei gleichzeitigem Entfall aller weiteren, distalen Segmente. Ebenso ist denkbar, die vorliegende Erfindung für oszillometrische oder tonometrische Messungen zu verwenden, bei denen ein hämodynamischer Verlauf unter Teilokklusion einer Arterie erfolgt, wenn dazu im Analogon (2) der Wert des Leitungswiderstands R am Windkesselelement (Fig. 1) des betreffenden Gefäßabschnitts entsprechend erhöht wird.

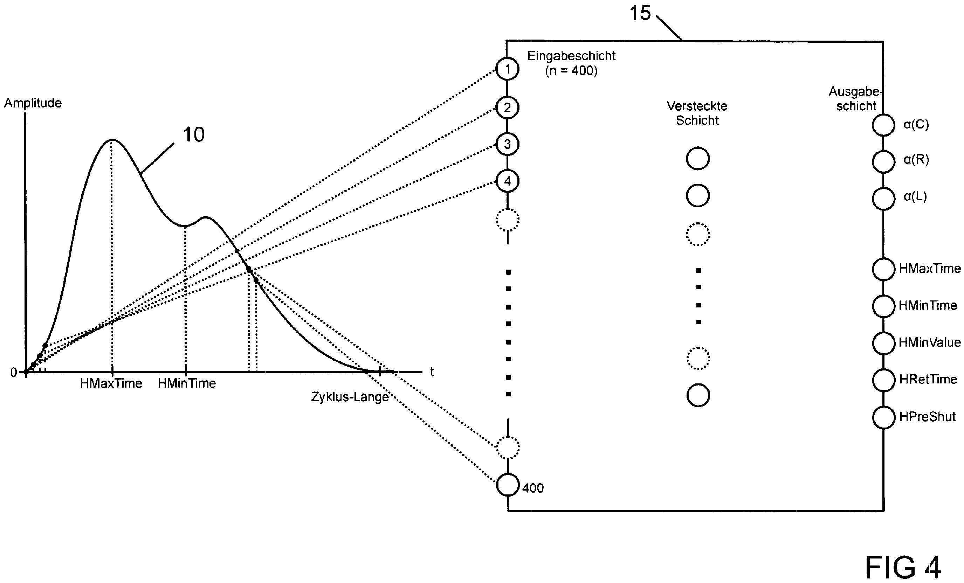

Die hämodynamischen Verläufe werden anschließend durch Signalverarbeitung so aufbereitet, dass diese in Form zeitlich digitalisierten Amplitudenwerte zur Verfügung stehen und dann nach Herzzyklen segmentiert werden, so dass hierdurch einzelne Pulsationen (10, Fig. 4) abgebildet werden. Durch Mittelwertbildung kann zusätzlich aus mehreren Einzelpulsationen (10) eine durchschnittliche Pulsation (10) gebildet werden, um die Robustheit des Verfahrens, z.B. gegenüber arrhythmischen Varianzen oder Wiederholungsmessungen zu erhöhen.

Zum Training des oben beispielhaft beschriebenen neuronalen Netzwerkes (15) werden dann gemäß des vorliegenden Ausführungsbeispiels die ersten Fünfhundert digitalisierten Amplitudenwerte einer so gewonnenen Pulsation (10) an einer korrespondierenden Anzahl von Eingängen eines Backpropagation-Netzes präsentiert, womit dann ein sogenanntes Überwachtes Lernen mit zufälliger Initialisierung der Neuronen-Gewichtung, eine Input-Unit mit Fünfhundert Eingängen, eine Hidden Unit sowie eine Output-Unit mit acht Ausgängen ermöglicht wird. Ergebnis sind die Parameter α(C), α(R), α(L), HMaxTime, HMinTime, HMinValue, HRetTime und HPreShut. Im vorliegenden Ausführungsbeispiel wurde eine praktikable Anzahl von Fünfhundert Amplitudenwerten gewählt, da einerseits die Anzahl der Eingangsdaten so niedrig wie möglich gewählt werden sollte, andererseits aber alle wesentlichen Merkmale eines Herzzyklus unter Berücksichtigung der minimal und maximal auftretenden Herzrate vollständig präsentiert werden müssen. Fünfhundert Amplitudenwerte entsprechen bei einer Signal-Abtastraste von 1 ms einem Zeitraum von 500 ms. Andere Sample-Anzahlen sind jedoch ebenfalls möglich, ebenso ist es im Sinne der vorliegenden Erfindung denkbar, dem erfindungsgemäßen Verfahren an dessen Eingängen Amplitudenwerte mehrerer, aufeinander folgender Einzelpulsationen (10) eines hämodynamischen Verlaufs gleichzeitig zu präsentieren.

Ein zweiter Aspekt dieser Erfindung zielt auf die Schaffung einer Vorrichtung, mit der die arterielle Compliance, der arterielle Leitungswiderstand und die Fließfähigkeit des Bluts automatisch, zuverlässig und reproduzierbar messbar sind.

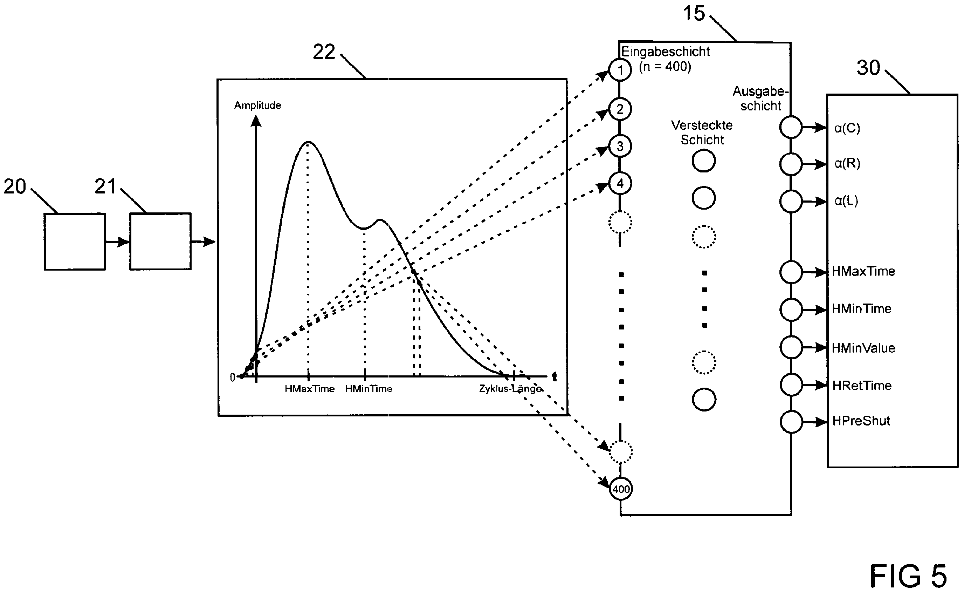

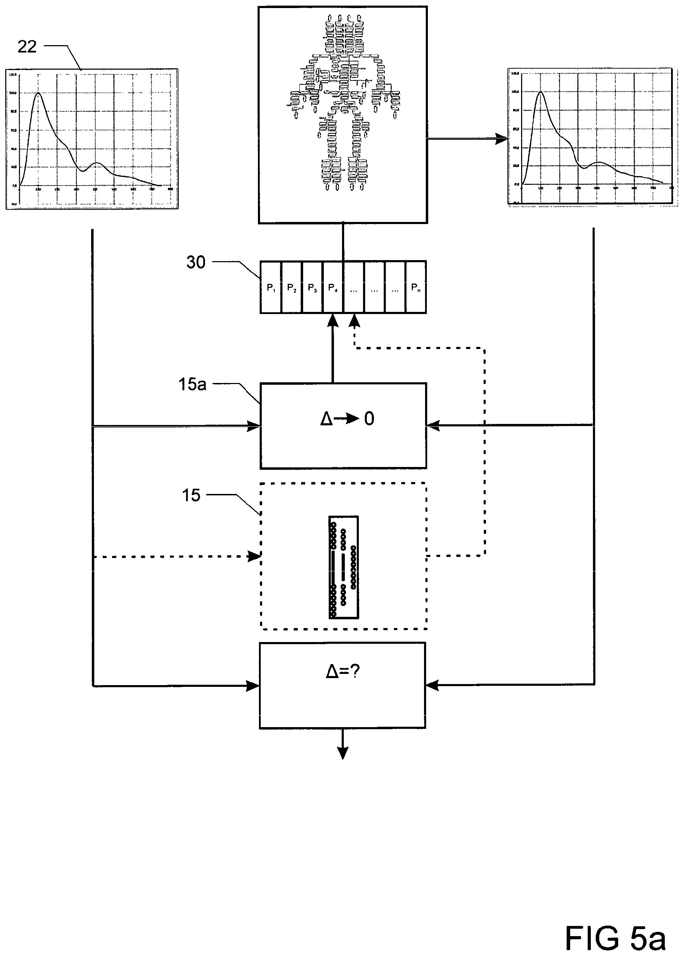

Fig. 5 und Fig. 5a illustrieren die Komponenten des vorliegenden Ausführungsbeispiels einer erfindungsgemäßen Vorrichtung. An einem Individuum werden mittels einer Sensorik (20) hämodynamische Verläufe abgenommen und einer Signalverarbeitungskette (21) zugeführt. Bei diesen hämodynamischen Verläufen kann es sich beispielsweise um einen sogenannten Druck-, Volumen- oder Flusspuls handeln, da sich die eingangs beschriebene elektro-hydraulische Analogie auf diese Größen bezieht.

Die so gewonnenen hämodynamischen Verläufe werden anschließend durch Signalverarbeitung (21) so aufbereitet, dass diese in Form zeitlich digitalisierter Amplitudenwerte zur Verfügung stehen und dann nach Herzzyklen segmentiert, so dass hierdurch einzelne Pulsationen (22) abgebildet werden. Durch Mittelwertbildung kann auch hier zusätzlich aus mehreren Einzelpulsationen (22) eine durchschnittliche Pulsation (22) gebildet werden, um die Robustheit des Verfahrens z.B. gegenüber arrhythmischen Varianzen oder Wiederholungsmessungen zu erhöhen. Darüber hinaus können mittels spezieller Analyseverfahren (z.B. Fourieranalyse) höherfrequente Artefakte in den Messungen eliminiert werden. Im Zuge der weiteren Verarbeitung werden die Amplitudenwerte der so gewonnenen Pulsation (22) der korrespondierenden Anzahl von Eingängen einer Vorrichtung (15) (Fig. 5) zugeführt, an dessen Ausgängen dann die gesuchten Werte der Parameter
- α(C) als Maß für die Compliance
- α(R) als Maß für den arteriellen Leitungswiderstand
- α(L) als Maß für die Massenträgheit des Bluts
- HMaxTime als Zeitpunkt des Maximums des Druck- oder Flusszyklus
- HMinTime als Zeitpunkt des Aortaherzklappenschlusses
- HMinValue
- HRetTime
- HPreShut
zur Verfügung stehen.

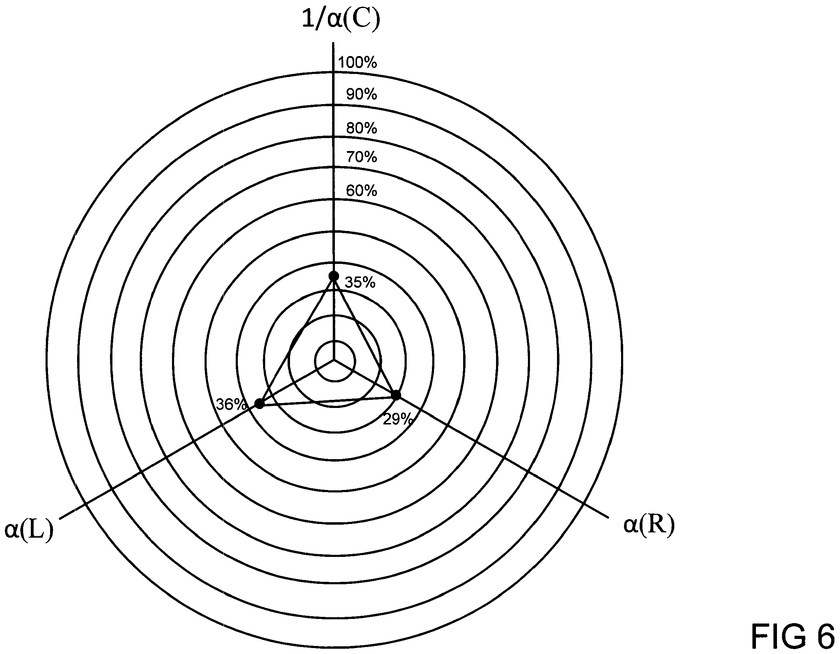

Ein wesentlicher Vorteil der Erfindung besteht darin, dass die gesuchten Parameter auf diese Weise sehr präzise ermittelt werden können. Die in Fig. 5 bzw. Fig. 5a dargestellten Komponenten können innerhalb einer mechanisch gekoppelten Apparatur untergebracht oder auf separate Sensor-, Rechen- und Anzeige-Systeme verteilt sein. Die Parameterwerte am Ausgang der Vorrichtung(15) können kurz- oder langfristig gespeichert und dadurch jederzeit mit einer Anzeigevorrichtung (30) dargestellt werden. Fig. 6 zeigt die vorteilhafte Darstellung der ermittelten, arteriellen Parameter Compliance α(C), Leitungswiderstand α(R) und Massenträgheit des Bluts α(L) in einem sogenannten Netz- oder Radar-Diagramm, wobei hier die sogenannte arterielle Steifheit als Kehrwert der Compliance 1/α(C) abgebildet ist.

Die in-vivo ermittelten hämodynamischen Druck-, Volumen- oder Fluss-Pulsverläufe können mittels Sensorik (20) invasiv oder nicht-invasiv aufgenommen werden, ohne von der grundlegenden Idee dieser Erfindung abzuweichen. Ein bereits zuvor beschriebener, wesentlicher Vorteil der Erfindung ist, dass die Ausführung, basierend auf der konkreten Gestaltung des Analogons 2, an verschiedene Messmethodiken anpassbar ist. So eignet sich eine Vorrichtung gem. Fig. 5 und Fig. 5a beispielsweise für invasive, aber auch für oszillometrische, suprasystolische Brachialmessungen, die Photoplethysmographie oder andere oszillometrische oder tonometrische Verfahren, die auf einer Teilokklusion verschiedener Gefäßabschnitte basieren, vorausgesetzt die Windkesselelemente (Fig. 1) des Analogons 2 wurden entsprechend den aus der in-vivo-Applikation des Sensors (20) resultierenden, physiologischen Auswirkungen angepasst.

In Fig. 5a werden beide Wege der Auswertung mittels neuronalem Netzwerk (15) oder Optimierungs-Algorithmus (15a) dargestellt ohne noch einmal auf die Sensorik (20) und die Signalverarbeitung (21) einzugehen. Die Anwendung des Optimierungs-Algorithmus bietet den Vorteil, dass die Ergebnisse sehr präzise und ohne vorheriges Training eines neuronalen Netzes erreicht werden. D.h., es werden in diesem Fall auch keine Trainingsdaten zur Verwendung des erfindungsgemäßen Verfahrens benötigt.

Figur 7 zeigt die Ausgabe ein Diagramm, in dem eine gemessene Pulsdruckkurve (1) und die zugehörige simulierte Pulsdruckkurve (2) übereinander dargestellt sind.

## Patentansprüche

1. Softwaregestütztes Verfahren zur Erfassung von arteriellen Parametern eines Menschen, wobei eine Pulskurve an dem Menschen gemessen wird, wobei ein elektro- hydraulisches Arterienmodell, ein Analogon (1), als Simulationsmodell gebildet wird, wobei die Pulskurve mit simulierten Pulskurven eines Simulationsmodells des Analogons (1) verglichen wird und verschiedene Parameter des Simulationsmodells solange variiert werden, bis die Pulskurve des Simulationsmodells mit der gemessenen Pulskurve in einer vorgesehenen Näherung übereinstimmt und wobei die arteriellen Parameter ausgegeben werden, **dadurch gekennzeichnet, dass**
das Simulationsmodell mit der Reihenschaltung mehrerer aus Kapazitäten C, ohmschen Widerständen R und Induktivitäten L bestehender Windkesselelemente gebildet wird, wobei 711 dieser Windkesselelemente mit auf 1 cm Windkesselelementsegmentlänge zurückgerechneten R, L und C Werten verwendet werden

2. Verfahren nach Anspruch 1, **dadurch gekennzeichnet, dass** die gemessene Pulskurve einer die Kurvenform erfassenden Berechnungseinheit zugeführt wird, und dass die Berechnungseinheit die Parameter eines Simulationsmodells einstellt.

3. Verfahren nach Anspruch 1 oder 2, **dadurch gekennzeichnet, dass** die Pulskurve gemessen wird, indem ein Manschettendruck bis zum Blutflussstillstand durch Okklusion der Arterien gesteigert wird, dass dieser Manschettendruck eine vorgegebene Anzahl an Herzzyklen aufrechterhalten wird und dass der Druckverlauf mehrerer Pulskurven in der Manschette gemessen wird und dass aus dem Druckverlauf mehrerer Pulskurven eine gemittelte Pulskurve erzeugt wird.

4. Verfahren nach einem der Ansprüche 1 - 3, **dadurch gekennzeichnet, dass** mit Hilfe des Simulationsmodells für den menschlichen Körper für jeden Arterienabschnitt proximal zum Messpunkt gelegen die Compliance, der arterielle Leitungswiderstand und die Massenträgheit des Bluts berechnet werden kann.

5. Vorrichtung zur Durchführung des Verfahrens nach einem der vorhergehenden Ansprüche, umfassend eine Berechnungseinheit mit:
einer Eingabeschnittstelle zur Eingabe der Daten einer Pulskurve,
einer Optimierungseinrichtung zur Bestimmung derjenigen Parameter eines Simulationsmodells, die eine optimale Übereinstimmung der Pulskurve mit einer simulierten Pulskurve ermöglichen und
einer Ausgabeschnittstelle zum Ausgeben der Parameter der nächstkommenden abgespeicherten Pulskurve hat.

6. Vorrichtung nach Anspruch 5, **dadurch gekennzeichnet, dass** die Berechnungseinheit ein neuronales Netzwerk ist.

7. Vorrichtung nach Anspruch 5 oder 6, **dadurch gekennzeichnet, dass** im Simulationsmodell des menschlichen Körpers die Arterien in einzelne Arterienabschnitte unterteilt sind und die Arterienabschnitte als elektrisches Ersatzschaltbild mittels Widerständen, Kapazitäten und Induktivitäten angenähert sind.

8. Vorrichtung nach einem der Ansprüche 5 bis 7, **dadurch gekennzeichnet, dass** eine Vorrichtung zur Messung der Pulskurve eine zur Anlage an dem menschlichen Körper vorgesehene Druckmanschette und eine Druckquelle zur Erzeugung eines den Stillstand des Blutflusses in den Arterien, insbesondere den Handgelenk-Arterien, ermöglichenden Drucks und zur Aufrechterhaltung dieses Drucks für eine vorgesehene Anzahl an Herzzyklen ausgebildet ist.

## Claims

1. Software-based method for detecting arterial parameters of a human being, wherein a pulse curve is measured on the human being, wherein an electro-hydraulic arterial model, an analogue (1), is formed as a simulation model, wherein the pulse curve is compared with simulated pulse curves of a simulation model of the analogue (1) and various parameters of the simulation model are varied until the pulse curve of the simulation model matches the measured pulse curve to an intended approximation, and wherein the arterial parameters are output,
**characterised in that**
the simulation model is formed by connecting multiple Windkessel elements consisting of capacitors C, ohmic resistors R and inductors L in series, wherein 711 of these Windkessel elements are used with R, L and C values back-calculated to a 1 cm Windkessel element segment length.

2. Method according to claim 1, **characterised in that** the measured pulse curve is fed to a calculation unit which detects the curve shape, and **in that** the calculation unit sets the parameters of a simulation model.

3. Method according to claim 1 or 2, **characterised in that** the pulse curve is measured by increasing a cuff pressure until blood flow stops by occluding the arteries, **in that** this cuff pressure is maintained for a predetermined number of cardiac cycles, and **in that** the pressure curve of multiple pulse curves is measured in the cuff, and **in that** an averaged pulse curve is generated from the pressure curve of multiple pulse curves.

4. Method according to any one of claims 1-3, **characterised in that** the compliance, the arterial conduction resistance and the mass inertia of the blood can be calculated for each arterial section proximal to the measuring point with the aid of the simulation model for the human body.

5. Device for carrying out the method according to any one of the preceding claims, comprising a calculation unit having:
an input interface for inputting the data from a pulse curve,
an optimisation apparatus for determining the parameters of a simulation model that enable an optimal match between the pulse curve and a simulated pulse curve, and
has an output interface for outputting the parameters of the next stored pulse curve.

6. Device according to claim 5, **characterised in that**
the calculation unit is a neural network.

7. Device according to claim 5 or 6, **characterised in that** in the simulation model of the human body the arteries are divided into individual arterial sections and the arterial sections are approximated as an electrical equivalent circuit diagram by means of resistors, capacitors and inductors.

8. Device according to any one of claims 5 to 7, **characterised in that** a device for measuring the pulse curve is formed of a pressure cuff intended to be placed on the human body and a pressure source for generating a pressure that allows the blood flow in the arteries, in particular the wrist arteries, to stop and for maintaining this pressure for a predetermined number of cardiac cycles.

## Revendications

1. Procédé assisté par logiciel pour la saisie de paramètres artériels d'un être humain, dans lequel une courbe de pouls est mesurée sur l'être humain, dans lequel un modèle artériel électro-hydraulique, un analogue (1), est formé en tant que modèle de simulation, dans lequel la courbe de pouls est comparée à des courbes de pouls simulées d'un modèle de simulation de l'analogue (1) et différents paramètres du modèle de simulation sont variés jusqu'à ce que la courbe de pouls du modèle de simulation coïncide avec la courbe de pouls mesurée dans une approximation prévue et dans lequel les paramètres artériels sont émis,
**caractérisé en ce que**
le modèle de simulation est formé avec le montage en série de plusieurs éléments de réservoir d'air constitués de capacités C, de résistances ohmiques R et d'inductances L, dans lequel 711 de ces éléments de réservoir d'air sont utilisés avec des valeurs R, L et C recalculées sur une longueur de segment d'élément de réservoir d'air de 1 cm.

2. Procédé selon la revendication 1, **caractérisé en ce que** la courbe de pouls mesurée est acheminée vers une unité de calcul saisissant la forme de courbe, et **en ce que** l'unité de calcul règle les paramètres d'un modèle de simulation.

3. Procédé selon la revendication 1 ou 2, **caractérisé en ce que** la courbe de pouls est mesurée **en ce qu'**une pression de brassard jusqu'à l'arrêt du flux sanguin par occlusion des artères est augmentée, **en ce que** cette pression de brassard est maintenue pendant un nombre prédéterminé de cycles cardiaques et **en ce que** l'évolution de la pression de plusieurs courbes de pouls est mesurée dans le brassard et **en ce qu'**une courbe de pouls moyennée est générée à partir de l'évolution de la pression de plusieurs courbes de pouls.

4. Procédé selon l'une quelconque des revendications 1 à 3, **caractérisé en ce que** la compliance, la résistance de conduction artérielle et l'inertie de masse du sang peuvent être mesurées pour chaque section d'artère située à proximité du point de mesure à l'aide du modèle de simulation pour le corps humain.

5. Dispositif pour la mise en oeuvre du procédé selon l'une quelconque des revendications précédentes, comprenant une unité de calcul avec :
une interface d'entrée pour l'entrée des données d'une courbe de pouls,
un appareil d'optimisation pour déterminer les paramètres respectifs d'un modèle de simulation qui permettent une concordance optimale de la courbe de pouls avec une courbe de pouls simulée et
une interface de sortie pour émettre les paramètres de la courbe de pouls enregistrée la plus proche.

6. Dispositif selon la revendication 5, **caractérisé en ce que** l'unité de calcul est un réseau neuronal.

7. Dispositif selon la revendication 5 ou 6, **caractérisé en ce que** dans le modèle de simulation du corps humain, les artères sont divisées en sections d'artère individuelles et les sections d'artère sont rapprochées en tant que schéma électrique équivalent au moyen de résistances, capacités et inductances.

8. Dispositif selon l'une quelconque des revendications 5 à 7, **caractérisé en ce qu'**un dispositif de mesure de la courbe de pouls est constitué d'un brassard de pression prévu pour être appliqué sur le corps humain et d'une source de pression pour générer une pression permettant l'arrêt du flux sanguin dans les artères, en particulier les artères du poignet, et pour maintenir cette pression pendant un nombre prévu de cycles cardiaques.
